**Europäisches Patentamt**

**European Patent Office**

**Office européen des brevets**

(19)

(11) Numéro de publication : **0 105 812
B1**

(12) # FASCICULE DE BREVET EUROPÉEN

(45) Date de publication du fascicule du brevet :
05.03.86

(51) Int. Cl.⁴ : **A 61 B 10/00**

(21) Numéro de dépôt : **83401941.6**

(22) Date de dépôt : **04.10.83**

(54) Appareil d'échotomographie d'organes externes, notamment des glandes mammaires.

(30) Priorité : **05.10.82 FR 8216663**

(43) Date de publication de la demande :
**18.04.84 Bulletin 84/16**

(45) Mention de la délivrance du brevet :
**05.03.86 Bulletin 86/10**

(84) Etats contractants désignés :
**AT BE CH DE GB IT LI LU NL SE**

(56) Documents cités :
**EP-A- 0 032 751
EP-A- 0 089 682
DE-A- 3 136 037
FR-A- 2 425 837
FR-A- 2 492 653
FR-A- 2 519 540
US-A- 4 063 097**

(73) Titulaire : **Franceschi, Claude
21, quai Alphonse Le Gallo
F-92100 Boulogne (FR)**

**Luizy, François
36, rue de la Félicité
F-75017 Paris (FR)**

**Luizy, Jean
Zodiac 2 Boulevard Front de Mer Hyeres Plage
F-83400 Hyeres (FR)**

**Vadrot, Dominique
16, rue M. Barthélémy
F-94120 Fontenay S/Bois (FR)**

**Vadrot, Martine
16, rue M. Barthélémy
F-94120 Fontenay S/Bois (FR)**

(72) Inventeur : **Franceschi, Claude
21, quai Alphonse Le Gallo
F-92100 Boulogne (FR)**
Inventeur : **Luizy, François
36, rue de la Félicité
F-75017 Paris (FR)**
Inventeur : **Luizy, Jean
Zodiac 2 Boulevard Front de Mer Hyeres Plage
F-83400 Hyeres (FR)**
Inventeur : **Vadrot, Dominique
16, rue M. Barthélémy
F-94120 Fontenay S/Bois (FR)**
Inventeur : **Vadrot, Martine
16, rue M. Barthélémy
F-94120 Fontenay S/Bois (FR)**

(74) Mandataire : **Peuscet, Jacques
3, Square de Maubeuge
F-75009 Paris (FR)**

Jouve, 18, rue St-Denis, 75001 Paris, France

## Description

La présente invention concerne l'échotomographie d'organes de petites dimensions, et elle concerne plus précisément un appareil d'échotomographie des glandes mammaires.

Les techniques d'échotomographie mettent en œuvre un faisceau ultra-sonique d'analyse émis dans une zone en éventail et déplacé successivement dans des plans différents afin de permettre une exploration complète de l'organe étudié.

Un appareil connu d'échotomographie des glandes mammaires comprend un récipient de grande dimension au fond duquel est monté un chariot mobile en translation. Une sonde d'échotomographie, portée par ledit chariot et formant un faisceau en éventail contenu dans un plan, est déplacée progressivement parallèlement à elle-même, dans une direction perpendiculaire au plan d'analyse. De cette manière, lorsque le récipient de l'appareil est rempli d'eau et lorsque les glandes mammaires d'une patiente sont disposées en suspension dans l'eau au-dessus de la sonde, l'appareil réalise des observations dans des plans parallèles, qui balayent la glande.

Cet appareil connu présente des inconvénients importants. En premier lieu, l'appareil est strictement spécialisé et son coût élevé est donc difficilement amortissable. En second lieu, aucun réglage en hauteur de l'appareillage n'est réalisable en fonction de la dimension des glandes mammaires : en conséquence, étant donné que la focalisation du faisceau est fixe, les images obtenues sont souvent floues et peu significatives. Il peut sembler à première vue facile de réaliser un mécanisme de réglage en hauteur de la sonde. En fait, cette caractéristique est cependant de réalisation assez difficile car l'ensemble comprenant la sonde et son chariot est disposé dans l'eau ; de plus, une telle modification entraînerait encore une augmentation du prix de l'appareillage.

L'invention concerne un appareil d'échotomographie utilisable pour les glandes mammaires ainsi que pour d'autres organes externes de petites dimensions, cet appareil ne présentant pas les inconvénients précités ; plus précisément, aucun mécanisme ne fonctionne sous l'eau dans l'appareil selon l'invention. En conséquence, cet appareil peut comprendre, sans augmentation excessive du prix de revient, de nombreux dispositifs de réglage, permettant notamment le réglage en distance de la sonde ou émetteur-récepteur d'ultra-sons par rapport à l'organe à étudier et, le réglage du plan d'examen par rotation autour d'un axe vertical et/ou autour d'un axe horizontal.

Dans cet appareil, la sonde émettrice-réceptrice est directement au contact de l'eau qui est aussi au contact de l'organe à analyser. Le réglage de la distance séparant la sonde de l'organe permet une focalisation optimale et ainsi l'obtention d'une image très nette.

En outre, les différentes possibilités de réglage permettent des examens de différents types et non un simple balayage par plans parallèles comme dans l'appareil connu. En conséquence, la direction du plan d'examen et la séquence d'exploration peuvent être modifiées en fonction de la structure particulière de l'organe étudié.

L'invention a donc pour objet un appareil d'échotomographie utilisable pour l'examen d'organes externes et notamment des glandes mammaires, cet appareil comprenant, sur un bâti, un récipient muni d'une ouverture destiné à contenir un liquide assurant une bonne transmission des ultra-sons et baignant l'organe à examiner, un émetteur-récepteur d'ultra-sons formant un faisceau d'examen en éventail plat qui se propage dans le récipient précité, ledit émetteur-récepteur étant déplacé par rapport à l'organe examiné au moyen d'un mécanisme à premier chemin circulaire de roulement permettant de faire tourner l'émetteur-récepteur par rapport au bâti autour de l'axe du premier chemin de roulement, et d'un mécanisme permettant de déplacer angulairement l'émetteur-récepteur par rapport au plan de l'ouverture, caractérisé par le fait que :

en premier lieu, le récipient est une cuvette comprenant une collerette périphérique circulaire rigide et un corps formé d'une membrane souple, ladite collerette comportant une lèvre périphérique qui forme déversoir pour le liquide contenu dans le récipient, lèvre sous laquelle est disposé un deuxième chemin circulaire de roulement qui supporte la cuvette, la membrane constituant le corps étant fixée de manière étanche par sa périphérie sur la collerette et par sa zone centrale sur l'émetteur-récepteur, de manière que la fenêtre de passage du faisceau de l'émetteur-récepteur soit directement au contact avec le liquide placé dans la cuvette, mais que ledit émetteur-récepteur soit disposé à l'extérieur de ladite cuvette ;

en second lieu, le bâti délimite un logement où est disposé le récipient, ledit logement étant entouré par le premier chemin circulaire de roulement, qui a sensiblement le même diamètre que le deuxième chemin de roulement de la collerette, une rigole entourant extérieurement le premier chemin de roulement du bâti et étant destinée à recevoir le liquide débordant au-dessus de la lèvre-déversoir de la collerette, des organes de roulement étant disposés entre les chemins de roulement du bâti et de la collerette ;

en troisième lieu, un mécanisme d'entraînement permet de faire tourner la cuvette, avec l'émetteur-récepteur, par rapport au bâti autour de l'axe commun des chemins de roulement.

Le mécanisme destiné à faire tourner la cuvette comporte avantageusement un moteur et une courroie crantée passant autour de la collerette, ladite courroie étant entraînée par le moteur.

L'appareil comporte avantageusement un mécanisme d'ajustement de la distance de l'émetteur-récepteur par rapport au centre de la collerette. Ce mécanisme d'ajustement comporte, de préférence, une suspension articulée sur la collerette autour d'un axe parallèle au plan de ladite collerette et passant par le centre de celle-ci, l'émetteur-récepteur et une commande du mécanisme d'ajustement étant montés sur ladite suspension. En outre, la suspension articulée est avantageusement constituée de deux tiges filetées libres en rotation et d'un organe muni d'écrous non rotatifs montés sur lesdites tiges, ledit organe portant l'émetteur-récepteur, les tiges portant une plaque fixe par rapport aux axes desdites tiges sur laquelle est disposé un moteur destiné à faire tourner en synchronisme les tiges filetées autour de leur axe.

Dans l'appareil selon l'invention, on préfère que le mécanisme permettant de déplacer angulairement l'émetteur-récepteur par rapport au plan de la collerette comporte un arceau solidaire de la collerette et disposé dans un plan perpendiculaire à l'axe d'articulation de la suspension, ce plan passant par le centre de la collerette, la plaque portée par les tiges de la suspension supportant le moteur qui commande la rotation de la suspension par rapport à l'arceau.

Dans un mode de réalisation préféré, l'appareil comprend des commutateurs de fin de course destinés à limiter la rotation de la cuvette à un angle inférieur à un tour et/ou des commutateurs de fin de course destinés à limiter la course du mécanisme d'ajustement et/ou du mécanisme permettant de déplacer angulairement l'émetteur-récepteur par rapport au plan de la collerette ; l'appareil comprend au moins deux tuyauteries dont l'une débouche dans la membrane de la cuvette et l'autre dans la rigole du bâti, la première tuyauterie étant alimentée en liquide par une pompe de circulation qui aspire ledit liquide dans un réservoir et la seconde tuyauterie renvoyant ledit liquide dans le réservoir précité ; l'appareil comprend une table d'appui solidaire du bâti et disposée au-dessus de la cuvette, cette table délimitant un orifice circulaire de diamètre inférieur au diamètre extérieur de la collerette.

Dans une réalisation avantageuse, l'émetteur-récepteur est une sonde d'échotomographie fixée de façon amovible dans un support qui assure l'étanchéité vis-à-vis du liquide contenu dans la cuvette, ledit support étant solidaire de la partie centrale de la membrane et étant constitué sensiblement comme indiqué dans la demande de brevet français FR-A-2 519 540.

Pour mieux faire comprendre l'objet de l'invention, on va en décrire maintenant, à titre d'exemple illustratif, un mode de réalisation et des variantes, représentés sur les dessins annexés. Sur ces dessins :

la figure 1 est une coupe schématique, selon I-I de la figure 3, d'un mode de réalisation d'appareil d'échotomographie selon l'invention ;

la figure 2 est une coupe agrandie du détail cerclé A de la figure 1 ;

la figure 3 est une coupe schématique selon III-III de la figure 1 ;

la figure 4 est analogue à la figure 3 mais représente l'appareil après déplacement de la sonde, selon une possibilité de réglage, et

la figure 5 est une vue de dessous de l'appareil de la figure 1.

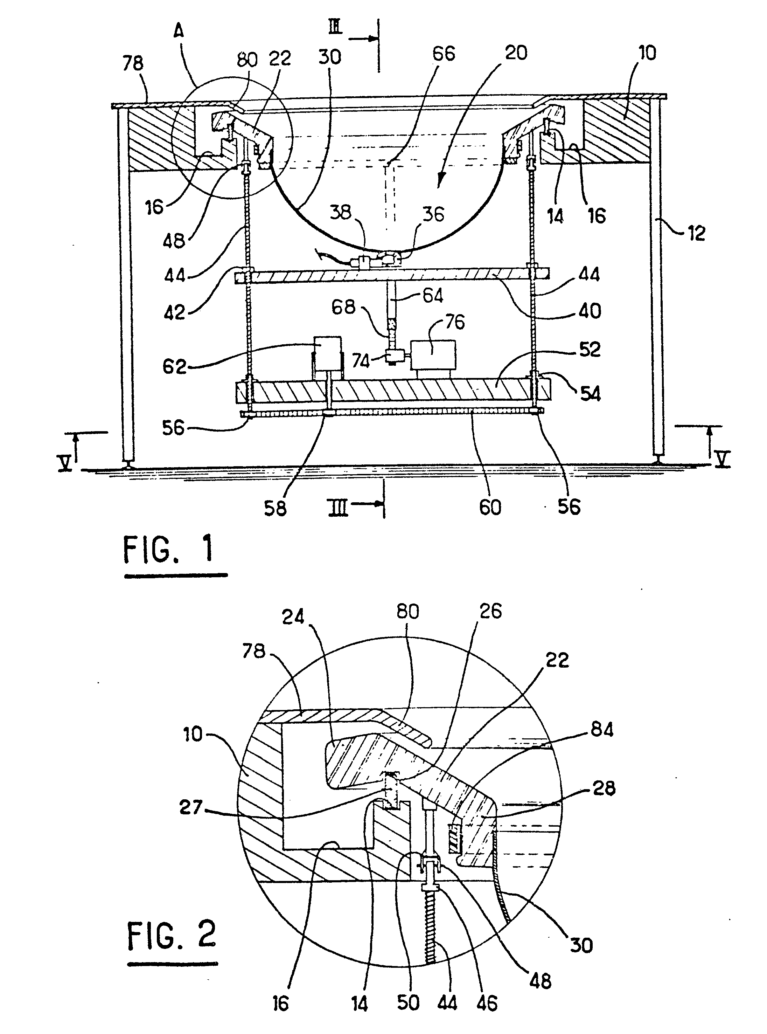

Les figures représentent un mode de réalisation d'appareil selon l'invention. Cet appareil comprend une table 10 de support montée sur quatre pieds 12. La table 10 délimite un orifice circulaire, dont le pourtour est entouré d'un premier chemin de roulement 14, clairement représenté sur la figure 2. Une rigole circulaire 16 est délimitée à l'extérieur du chemin de roulement 14 et une tuyauterie souple 18 communique par un orifice du fond de la table 10 avec la rigole 16.

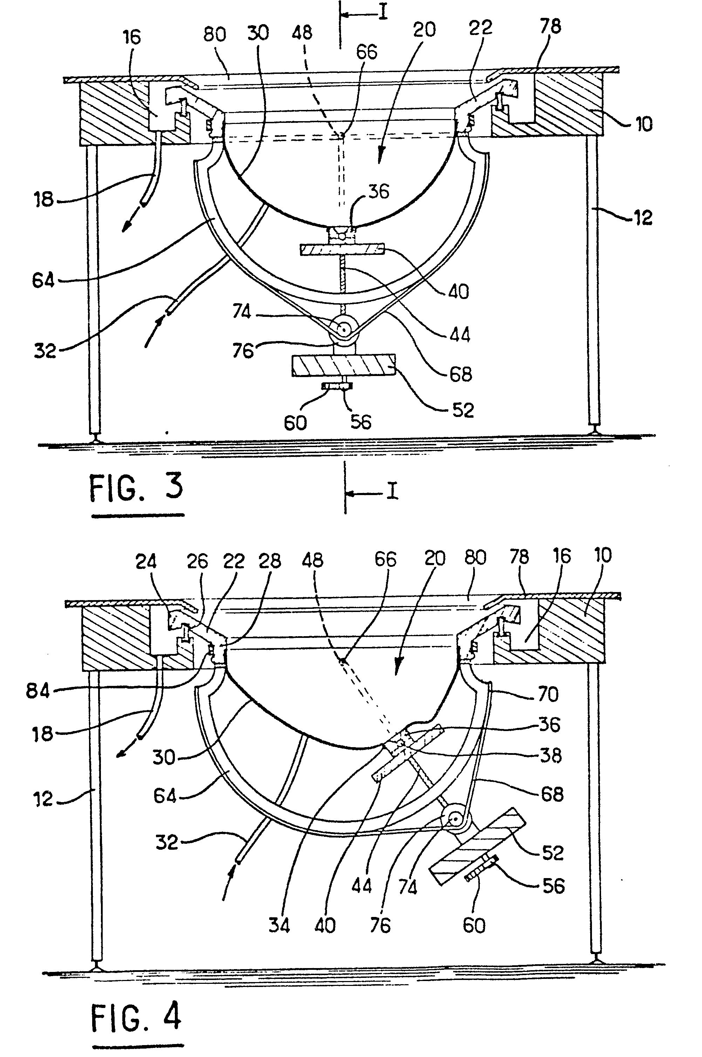

La table 10 porte un ensemble mobile formant une cuvette repérée par la référence générale 20. Cette cuvette comporte une collerette 22 qui, comme représenté sur la figure 2, délimite une lèvre externe 24 légèrement inclinée vers l'extérieur au-dessus de la rigole 16. La face inférieure de la collerette, à proximité de la lèvre 24, forme un deuxième chemin de roulement 26 si bien que des organes roulants 27, par exemple des galets, peuvent supporter la collerette 22 au-dessus du chemin de roulement 14 tout en permettant la rotation de la collerette. Vers l'intérieur, la collerette se termine par une partie sensiblement cylindrique 28 qui, à sa face interne, est solidaire d'une membrane souple 30, par exemple formée de caoutchouc néoprène. Cette membrane 30 communique avec une tuyauterie 32 d'entrée d'eau. Elle est solidaire, dans sa partie centrale, d'un manchon 34 qui est serré de façon étanche contre le support 36 d'une sonde à ultra-sons 38, constituant un émetteur-récepteur destiné à former un faisceau en éventail plat. Cette sonde peut être facilement retirée de son support lorsqu'elle doit être changée. Il s'agit d'une sonde bien connue dans la technique et on ne la décrira donc pas en détail ; on pourra se référer, pour plus de précision concernant la sonde 38 et son support 36, à la demande de brevet français FR-A-2 519 540. Comme l'indiquent les figures 1 et 3 qui sont des coupes à 90° l'une de l'autre, l'éventail ultrasonore projeté par la sonde 36 s'étale dans le plan de la figure 3, c'est-à-dire qu'il est perpendiculaire au plan de la figure 1.

La cuvette 20 est alimentée en eau par la tuyauterie 32 ; l'eau remplit la cuvette 20 jusqu'au niveau de la lèvre 24 qui forme un déversoir ; l'eau tombe dans la rigole 16 d'où elle est évacuée par la tuyauterie 18 vers un réservoir (non représenté) où elle est repompée par une pompe de circulation (non représentée) et renvoyée dans la tuyauterie 32.

Le support 36 de la sonde 38 n'est pas suspendu à la membrane souple 30 qui est relativement fragile : il est monté sur un organe 40 formant une platine qui porte, à ses deux extrémités, des écrous 42 qui ne peuvent pas tourner par rapport à la platine 40 et dans lesquels passent deux tiges filetées identiques 44 fixées sous la collerette 42.

Plus précisément, la figure 2 montre comment les tiges filetées 44 sont fixées à la collerette 22.

Chaque tige 44 a son extrémité engagée dans une chape 46 dont la partie supérieure est articulée sur un axe transversal 48 retenu par un étrier 50 qui dépasse sous la collerette 22. De cette manière, lorsque la tige 44 est mise en place dans la chape 46, l'axe 48 permet un pivotement autour d'un axe horizontal passant par le centre 66 de la collerette 22 mais la tige 44 peut pivoter librement autour de son axe par rapport à la chape 46.

A leur partie inférieure, les tiges 44 peuvent tourillonner librement dans des manchons 54, qui sont portés par une plaque 52 parallèle à la platine 40. Les extrémités inférieures des tiges 44, au-dessous de la plaque 52, portent des poulies 56 sur lesquelles passe une courroie crantée 60, qui est entraînée par un pignon 58 monté sur l'arbre d'un moteur 62.

Lorsque le moteur 62 est alimenté, il fait tourner le pignon 58 et les pignons 56 si bien que les vis 44 tournent en synchronisme à la même vitesse et provoquent le déplacement des écrous 42 le long des tiges 44. La platine 40 se déplace donc verticalement sur la figure 1 et permet un réglage de la distance de la sonde par rapport au centre 66 de la collerette 22. Ce déplacement en hauteur est possible car la membrane 30 est souple et peut donc se déformer.

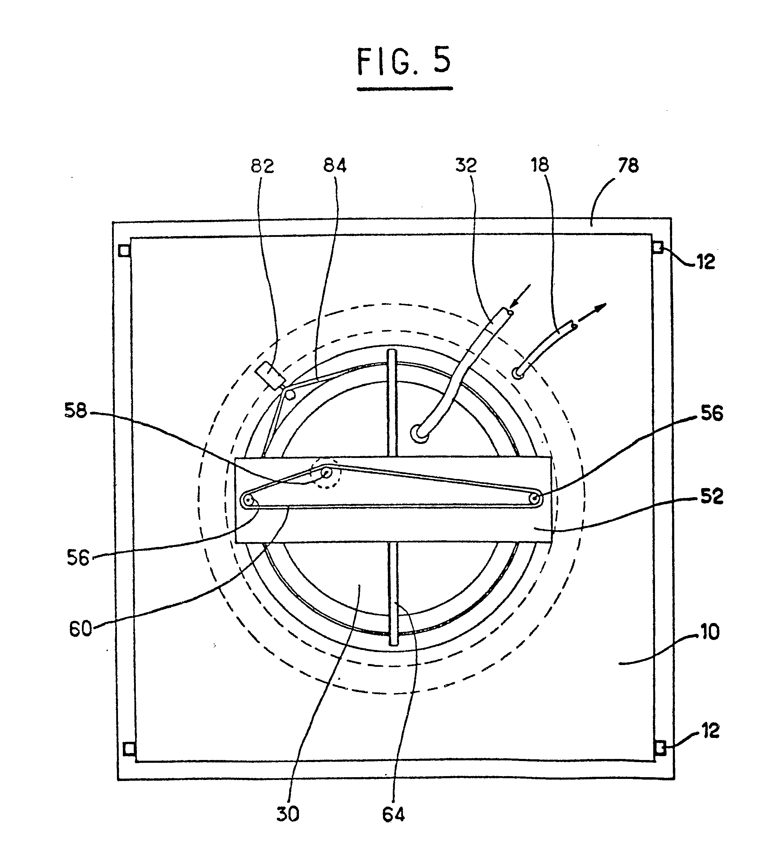

Les figures 3 et 4 montrent qu'un arceau 64 en demi-cercle, centré sur le centre 66 de la collerette 22, est solidaire de la partie inférieure de ladite collerette 22. Cet arceau 64, formé d'une matière rigide, est enveloppé partiellement par une courroie 68 fixée à ses extrémités 70 sur l'arceau 64. La longueur de la courroie 68 est supérieure à celle de l'arceau si bien qu'elle peut s'écarter de celui-ci et permet le passage d'un pignon 74 monté sur l'arbre d'un moteur 76 ; ce dernier est porté par la plaque 52. La platine 40 et la plaque 52 sont disposées de part et d'autre de l'arceau 64.

Lorsque le moteur 76 tourne dans un sens ou dans l'autre, il fait tourner la poulie 74, qui se déplace le long de la courroie 68 et provoque ainsi le pivotement de la suspension comprenant les tiges 44 et les plaques 40, 52. Cette suspension est articulée autour des axes 48, dont la direction passe par le centre 66 de l'arceau 64 et de la collerette 22.

Comme l'indique la description qui précède, la sonde 38 peut être déplacée en distance par rapport au centre 66 et en rotation autour d'un axe horizontal passant par le centre 66. L'appareil selon l'invention permet encore un autre mouvement. Une plaque d'appui 78 est fixée sur la face supérieure de la table 10 et se termine par un bord incliné 80 du côté de la cuvette 20 ; au droit du bord 80, la table 10 porte sur sa face inférieure, un moteur 82 qui entraîne une courroie crantée 84 passant à la périphérie de la partie cylindrique 28 de la collerette. De cette manière, l'entraînement en rotation du moteur 82 provoque un entraînement en rotation de l'ensemble de la cuvette 20, avec toute la suspension soutenue par la collerette 22.

Bien qu'on ne l'ait pas représenté, l'appareil comprend des commutateurs de fin de course destinés à empêcher que chacun des trois déplacements possibles soit excessif. Des commutateurs de fin de course portés par la table 10 sont déclenchés par des doigts dépassant de la collerette afin de limiter la rotation de la collerette dans une plage angulaire de 270°. De même, des commutateurs de fin de course sont montés sur l'arceau et sont déclenchés par des doigts solidaires de la suspension, par exemple de la plaque 52, afin de limiter le déplacement angulaire autour du centre 66 à 60° de part et d'autre de la position centrale représentée sur la figure 3. Enfin, des commutateurs de fin de course sont montés sur les tiges 44 et sont déclenchés par la plaque 40 afin que celle-ci ne puisse pas trop se rapprocher de la collerette 22 ou de la plaque 52.

En outre, les différents moteurs et la sonde sont évidemment alimentés par les lignes et câbles nécessaires que l'on n'a pas représentés. Cette alimentation ne pose pas de problème car la rotation de la cuvette est limitée à moins d'un tour. Il est préférable que tous les organes électriques soient alimentés en basse tension, par exemple en 12 V. En outre, la rotation limitée évite l'application de contraintes excessives sur la tuyauterie 32.

La comparaison des figures 3 et 4 montre les déformations que peut subir la membrane souple 30. Ces déformations sont bien inférieures à celles que peuvent supporter normalement de telles membranes si bien que leur longévité n'est pas affectée.

L'appareil décrit précédemment permet des examens d'échotomographie suivant diverses techniques. En effet, l'éventail ultra-sonique peut rester dans un plan vertical tournant autour du centre 66 par rotation de la cuvette. Il peut aussi être incliné par rapport à la verticale par commande du moteur 76 et les plans d'examen peuvent alors être radiaux par rapport à l'axe horizontal 48 de basculement. Des combinaisons de ces divers balayages donnent une grande souplesse d'utilisation de l'appareil.

Les images obtenues avec l'appareil ont une très bonne résolution car la sonde peut être focalisée de façon optimale par rapport à l'organe examiné grâce au réglage commandé par le moteur 62.

Lors du fonctionnement, une patiente allongée sur un lit place sa poitrine au contact de la plaque supérieure 78, sa glande mammaire étant disposée dans la cuvette 20. La distance de la sonde 38 est réglée alors de manière optimale, et l'examen est réalisé alors que de l'eau à température choisie circule dans la cuvette 20. La configuration de l'appareil permet l'utilisation d'un faible volume d'eau de circulation si bien que la pompe de circulation peut être de petite dimension.

Ainsi, l'invention concerne un appareil de petite dimension, fiable, robuste, de manipulation aisée et de faible coût permettant cependant des examens très précis et de type très divers.

Il est bien entendu que l'appareil ci-dessus décrit pourra donner lieu à toute modification désirable, sans sortir pour cela du cadre de l'invention définie dans les revendications jointes.

**Revendications**

1. Appareil d'échotomographie utilisable pour l'examen d'organes externes et notamment des glandes mammaires, cet appareil comprenant, sur un bâti (20), un récipient (10) muni d'une ouverture, destiné à contenir un liquide assurant une bonne transmission des ultra-sons et baignant l'organe à examiner, un émetteur-récepteur d'ultra-sons (38) formant un faisceau d'examen en éventail plat qui se propage dans le récipient précité, ledit émetteur-récepteur étant déplacé par rapport à l'organe examiné au moyen d'un mécanisme à premier chemin circulaire de roulement (14) permettant de faire tourner l'émetteur-récepteur par rapport au bâti autour de l'axe du premier chemin de roulement, et d'un mécanisme (44, 48, 64, 68, 76) permettant de déplacer angulairement l'émetteur-récepteur par rapport au plan de l'ouverture, caractérisé par le fait que :

en premier lieu, le récipient est une cuvette (20) comprenant une collerette périphérique circulaire rigide (22) et un corps (30) formé d'une membrane souple, ladite collerette (22) comportant une lèvre (24) périphérique qui forme déversoir pour le liquide contenu dans le récipient, lèvre sous laquelle est disposé un deuxième chemin circulaire de roulement (26) qui supporte la cuvette, la membrane constituant le corps (30) étant fixée de manière étanche par sa périphérie sur la collerette (22) et par sa zone centrale sur l'émetteur-récepteur (38), de manière que la fenêtre de passage du faisceau de l'émetteur-récepteur (38) soit directement au contact avec le liquide placé dans la cuvette, mais que ledit émetteur-récepteur (38) soit disposé à l'extérieur de ladite cuvette ;

en second lieu, le bâti (10) délimite un logement où est disposé le récipient (20), ledit logement étant entouré par le premier chemin circulaire de roulement (14) qui a sensiblement le même diamètre que le deuxième chemin de roulement (26) de la collerette (22), une rigole (16) entourant extérieurement le premier chemin de roulement (14) du bâti et étant destinée à recevoir le liquide débordant au-dessus de la lèvre-déversoir (24) de la collerette (22), des organes de roulement (27) étant disposés entre les chemins de roulement (14, 26) du bâti (10) et de la collerette (22) ;

en troisième lieu, un mécanisme (82, 84) d'entraînement permet de faire tourner la cuvette (20), avec l'émetteur-récepteur, par rapport au bâti autour de l'axe commun des chemins de roulement (14, 26).

2. Appareil selon la revendication 1, caractérisé par le fait que le mécanisme destiné à faire tourner la cuvette (20) comporte un moteur (82) et une courroie crantée (84) passant autour de la collerette (22).

3. Appareil selon l'une des revendications 1 ou 2, caractérisé par le fait qu'il comprend un mécanisme (40, 42, 44, 62) d'ajustement de la distance de l'émetteur-récepteur (38) par rapport au centre de la collerette (22).

4. Appareil selon la revendication 3, caractérisé par le fait que le mécanisme d'ajustement comporte une suspension (40, 44, 52) articulée sur la collerette (22) autour d'un axe (48) parallèle au plan de la collerette (22) et passant par le centre (66) de celle-ci, l'émetteur-récepteur (38) et une commande (62) du mécanisme d'ajustement étant montés sur ladite suspension.

5. Appareil selon la revendication 4, caractérisé par le fait que la suspension articulée comporte deux tiges filetées (44) libres en rotation et un organe (40) muni d'écrous non rotatifs (42) montés sur les tiges (44), l'organe (40) portant l'émetteur-récepteur (38), les tiges (44) portant une plaque (52) fixe par rapport aux axes desdites tiges sur laquelle est disposé un moteur (62) destiné à faire tourner en synchronisme les tiges filetées (44) autour de leurs axes.

6. Appareil selon la revendication 5, caractérisé par le fait que le mécanisme permettant de déplacer angulairement l'émetteur-récepteur (38) par rapport au plan de la collerette (22) comporte un arceau (64) solidaire de la collerette (22) et disposé dans un plan perpendiculaire à l'axe (48) de la suspension, ce plan passant par le centre de la collerette (22), la plaque (52) portée par les tiges (44) de la suspension supportant un moteur (76) qui commande la rotation de la suspension par rapport à l'arceau (64).

7. Appareil selon l'une des revendications 1 à 6, caractérisé par le fait qu'il comprend des commutateurs de fin de course destinés à limiter la rotation de la cuvette (20) à un angle inférieur à un tour et/ou des commutateurs de fin de course destinés à limiter la course du mécanisme d'ajustement (40, 42, 44, 62) et/ou du mécanisme (48, 64, 68, 76) permettant de déplacer angulairement l'émetteur-récepteur (38) par rapport au plan de la collerette (22).

8. Appareil selon l'une des revendications 1 à 7, caractérisé par le fait qu'il comprend au moins deux tuyauteries (18, 32) dont l'une (32) débouche dans la membrane (30) de la cuvette (20) et l'autre (18) dans la rigole (16) du bâti, la première tuyauterie (32) étant alimentée en liquide par une pompe de circulation, qui aspire ledit liquide dans un réservoir et la seconde tuyauterie (18) renvoyant ledit liquide dans le réservoir précité.

9. Appareil selon l'une des revendications 1 à 8, caractérisé par le fait qu'il comprend une table d'appui (78) solidaire du bâti et disposée au-dessus de la cuvette, cette table délimitant un orifice circulaire de diamètre inférieur au diamètre extérieur de la collerette (22).

10. Appareil selon l'une des revendications 1 à 9, caractérisé par le fait que l'émetteur-récepteur (38) est une sonde d'échotomographie fixée de façon amovible dans un support (36) qui assure l'étanchéité vis-à-vis du liquide contenu dans la cuvette (20), ledit support étant solidaire de la partie centrale de la membrane (30).

## Claims

1. Echotomographic apparatus usable for examination of external organs, and notably the breasts, said apparatus comprising, on a stand (10) a container (30) provided with an opening intended to contain a liquid capable of enhancing transmission of ultrasonic emission and in which the organ to be examined is immersible, an ultrasonic emitter-receiver (32) generating an examination beam in the form of a flat fan which propagates into said container, said emitter-receptor being displaced relative to the organ to be examined by means of a mechanism (14) having a first circular rolling path enabling the emitter-receptor to turn relative to the stand about the axis of symmetry of the first rolling path, and a mechanism (44, 48, 64, 68, 76) permitting the emitter-receptor to displace angularly with respect to the plane of the opening, characterised by the fact that :

firstly, the container is a bowl (20) comprising a rigid circular peripheral rim (22) and a body (30) formed of a flexible membrane, said rim (22) comprising a peripheral lip (24) which forms an overflow for the liquid in the container, a second circular rolling path (26) being disposed under said lip and supporting the bowl, the membrane comprising the body (30) being fixed sealingly at its periphery to the rim (22) and by its central zone to the emitter-receiver (38) such that the window for the beam from the emitter-receiver (38) may be directly in contact with the liquid placed in the bowl but that said emitter-receiver (38) may be disposed outside said bowl,

secondly, the stand (10) defines a recess in which the container (20) is disposed, said recess being surrounded by the first circular rolling path (14) which has substantially the same diameter as the second rolling path (26) of the rim (32), a trough (16) externally surrounding the first rolling path (14) of the stand and being intended to receive liquid flowing over the overflow lip (24) of the rim (22) rolling members (27) being disposed between the rolling paths (14, 26) of the stand (10) and of the rim (22) ;

thirdly, a transmission mechanism (82, 84) enabling the bowl (20) to turn with the emitter-receiver relative to the stand around the common axis of the rolling paths (14, 26).

2. Apparatus according to claim 1, characterised by the fact that the mechanism intended to turn the bowl (20) comprises a motor (82) and a toothed belt (84) passing around the rim (22).

3. Apparatus according to claim 1 or claim 2, characterised by the fact that it comprises a mechanism (40, 42, 44, 62) for adjusting separation of the emitter-receiver (38) from the centre of the rim (22).

4. Apparatus according to claim 3, characterised by the fact that the adjustement mechanism comprises a suspension (40, 44, 52) articulated to the rim (22) around an axis (48) parallel to the plane of the rim (22) and passing through the centre (66) of the latter, the emitter-receiver (38) and a control (62) for the adjustment mechanism being mounted on said suspension.

5. Apparatus according to claim 4, characterised by the fact that the articulated suspension comprises two freely rotatable threaded rods (44) and a member (40) provided with non-rotatable nuts (42) mounted on the rods (44), the member (40) carrying the emitter-receiver (38), and the rods (44) carrying a plate (52) which is fixed relative to the axes of said rods and on which is mounted a motor (62) adapted to turn the threaded rods (44) synchronously around their oww axes.

6. Apparatus according to claim 5, characterised by the fact that the mechanism for angularly displacing the emitter-receiver (38) relative to the plane of the rim (22) comprises an arcuate member (64) fixed to the rim (22) and disposed in a plane perpendicular to the axis (48) of the suspension, this plane passing through the centre of the rim (22), the plate (52) carried by the rods (44) of the suspension supporting a motor (76) which controls rotation of the suspension relative to the arcuate member (64).

7. Apparatus according to any one of claims 1 to 6, characterised by the fact that it comprises limit switches intended to limit rotation of the bowl (20) to an angle less than one revolution, and/or limit switches intended to limit the travel of the adjustment mechanism (40, 42, 44, 62) and/or of the mechanism (48, 64, 68, 76) permitting angular displacement of the emitter-receiver (38) relative to the plane of the rim (22).

8. Apparatus according to any one of claims 1 to 7, characterised by the fact that it comprises at least two conduits (18, 32) one of which (32) opens into the membrane (30) of the bowl (20) and the other (18) into the channel (16) of the stand, said first conduit (32) being fed with liquid by a circulation pump which extracts said liquid from a reservoir and the second conduit (18) returns said liquid into said reservoir.

9. Apparatus according to any one of claims 1 to 8, characterised by the fact that it comprises a support table (78) fixed to the stand and disposed above the bowl, said table defining a circular orifice having a diameter less than the exterior diameter of the rim (22).

10. Apparatus according to any one of claims 1 to 9, characterised by the fact the emitter-receiver (38) is an echotomographic sensor removably fixed in a support (36) which ensures sealing relative to the liquid contents of the bowl (20), said support being fixed to the central part of the membrane (30).

## Patentansprüche

1. Echotomographievorrichtung für die Untersuchung von äußeren Organen und insbesondere von Brustdrüsen, bei welcher auf einem Rahmen (10) ein mit einer Öffnung versehener Behälter (20), der für die Aufnahme einer Flüssigkeit bestimmt ist, die eine gute Ultraschallübertragung gewährleistet und das zu untersuchende Organ benetzt, und ein Ultraschallsender-Empfänger (38) vorgesehen ist, der ein Untersuchungsbündel in Form eines ebenen Fächers bildet, das sich in dem genannten Behälter fortpflanzt, wobei der Sender-Empfänger bezüglich des untersuchten Organs mit Hilfe eines ersten kreisförmigen Laufschienenmechanismus (14), der ein Drehen des Sender-Empfängers bezüglich des Rahmens um die Achse der ersten Laufschiene ermöglicht, und mit Hilfe eines Mechanismus (44, 48, 64, 68, 76) verschiebbar ist, der eine Winkelverschiebung des Sender-Empfängers bezüglich der Ebene der Öffnung erlaubt, dadurch gekennzeichnet, daß

erstens der Behälter eine Küvette (20) mit einem starren kreisförmigen Umfangsbund (22) und einem Körper (30) ist, der von einer weichen Membran gebildet wird, wobei der Bund (22) eine Umfangslippe (24) aufweist, die einen Überlauf für die in dem Behälter vorhandene Flüssigkeit bildet, und unter der eine zweite kreisförmige Laufschiene (26), welche die Küvette trägt, angeordnet ist, die den Körper (30) bildende Membran abdichtend mit ihrem Umfang an dem Bund (22) und mit ihrem zentralen Bereich an dem Sender-Empfänger (38) befestigt ist, so daß das Fenster für den Durchgang des Bündels des Sender-Empfängers (38) direkt in Kontakt mit der Flüssigkeit steht, die in der Küvette angeordnet ist, jedoch der Sender-Empfänger (38) außerhalb der Küvette angeordnet ist ;

zweitens der Rahmen (10) einen Sitz begrenzt, in welchen der Behälter (20) angeordnet ist, wobei der Sitz von der ersten kreisförmigen Laufschiene (14) umgeben ist, die im wesentlichen den gleichen Durchmesser wie die zweite Laufschiene (26) des Bundes (22) hat, wobei die erste Laufschiene (14) des Rahmens außen von einer Rinne (16) umgeben ist, für die die Aufnahme der Flüssigkeit bestimmt ist, die über die Überlauflippe (24) des Bundes (22) hinüberläuft, und zwischen den Laufschienen (14, 26) des Rahmens (10) und des Bundes (22) Lauforgange (27) angeordnet sind, und

drittens ein Mitnahmemechanismus (82, 84) das Drehen der Küvette (20) mit dem Sender-Empfänger bezüglich des Rahmens um die gemeinsame Achse der Laufschienen (14, 26) ermöglicht.

2. Vorrichtung nach Anspruch 1, dadurch gekennzeichnet, daß der für das Drehen der Küvette (20) bestimmte Mechanismus einen Motor (82) und einen Zahnriemen (84) aufweist, der um den Bund (22) herumgeht.

3. Vorrichtung nach Anspruch 1 oder 2, dadurch gekennzeichnet, daß sie einen Mechanismus (40, 42, 44, 62) zur Einstellung des Abstands des Sender-Empfängers (38) bezüglich der Mitte des Bundes (22) aufweist.

4. Vorrichtung nach Anspruch 3, dadurch gekennzeichnet, daß der Mechanismus für die Einstellung eine Aufhängung (40, 44, 52) aufweist, die an dem Bund (22) um eine Achse (48) parallel zur Ebene des Bundes (22) und durch dessen Mitte (66) gehend angelenkt ist, wobei der Sender-Empfänger (38) und eine Steuerung (62) des Einstellmechanismus an dieser Aufhängung angebracht sind.

5. Vorrichtung nach Anspruch 4, dadurch gekennzeichnet, daß die angelenkte Aufhängung zwei drehungsfreie Gewindestangen (44) und ein Organ (40) aufweist, das mit nichtdrehenden, auf den Stangen (44) sitzenden Muttern (42) versehen ist, wobei das Organ (40) den Sender-Empfänger (38) trägt und die Stangen (44) eine Platte (52) halten, die bezüglich der Achsen der genannten Stangen festliegt, auf der ein Motor (62) angeordnet ist, der dazu bestimmt ist, die Gewindestangen (44) synchron um ihre Achsen zu drehen.

6. Vorrichtung nach Anspruch 5, dadurch gekennzeichnet, daß der die Winkelverschiebung des Sender-Empfängers (38) bezüglich der Ebene des Bundes (22) ermöglichende Mechanismus einen am Bund (22) festgelegten Bogen (64) aufweist, der in einer Ebene senkrecht zur Achse (48) der Aufhängung angeordnet ist, wobei diese Ebene durch die Mitte des Bundes (22) geht und die von den Stangen (44) der Aufhängung getragene Platte (52) einen Motor (76) trägt, der die Drehung der Aufhängung bezüglich des Bogens (64) steuert.

7. Vorrichtung nach einem der Ansprüche 1 bis 6, dadurch gekennzeichnet, daß sie Endschalter, die für die Begrenzung der Drehung der Küvette (20) um einen Winkel bestimmt sind, der kleiner als eine Umdrehung ist, und/oder Endschalter aufweist, die für die Begrenzung der Bahn des Einstellmechanismus (40, 42, 44, 62) und/oder des Mechanismus (48, 64, 68, 76) bestimmt sind, der die Winkelverschiebung des Sender-Empfängers (38) bezüglich der Ebene des Bundes (22) ermöglicht.

8. Vorrichtung nach einem der Ansprüche 1 bis 7, dadurch gekennzeichnet, daß sie wenigstens zwei Rohrleitungen (18, 32) aufweist, von denen die eine (32) in die Membran (30) der Küvette (20) und die andere (18) in die Rinne (16) des Rahmens mündet, wobei die erste Rohrleitung (32) mit Flüssigkeit von einer Umwälzpumpe gespeist wird, die die Flüssigkeit in einem Speicher ansaugt, während die zweite Rohrleitung (18) die Flüssigkeit in den genannten Speicher zurückführt.

9. Vorrichtung nach einem der Ansprüche 1 bis 8, dadurch gekennzeichnet, daß sie einen rahmenfesten Auflagetisch (78) aufweist, der über der Küvette angeordnet ist und eine kreisförmige Öffnung mit einem Durchmesser umgibt, der kleiner ist als der Außendurchmesser des Bundes (22).

10. Vorrichtung nach einem der Ansprüche 1 bis 9, dadurch gekennzeichnet, daß der Sender-Empfänger (38) eine Echotomographiesonde ist, die beweglich in einer Halterung (36) festgelegt ist, welche die Abdichtung gegenüber der Flüssigkeit in der Küvette (20) gewährleistet, wobei die Halterung fest an dem Mittelteil der Membrane (30) gehalten ist.

FIG. 1

FIG. 2

0 105 812

FIG. 3

FIG. 4

2

# FIG. 5